# EUROPEAN PATENT APPLICATION

(11) **EP 1 543 837 A1**
(43) Date of publication of application: **22.06.2005**
(21) Application number: 03028826.0
(22) Date of filing: 15.12.2003
(51) Int. Cl.: A61K 39/21, C07K 14/155, C07K 14/145, C12N 15/867

(54) **Virus-like particle (VLP) as vaccine**

(71) Applicant: Ruhr-Universität Bochum, 44801 Bochum (DE)
(72) Inventor: Überla, Klaus, Prof.Dr., 45529 Hattingen (DE); Kuate, Seraphin, Dr., 44791 Bochum (DE)
(74) Representative: Elbel, Michaela

(57) **Abstract**

The present invention relates to the use of a virus-like particle (VLP) for the preparation of a composition for immunization against an immunodeficiency virus, wherein said VLP is formed by at least a part of the viral protein Gag, said VLP comprises an envelope with at least one viral protein or a part thereof and at least one heterologous protein, said VLP is replication defective, and the at least one nucleic acid coding for said VLP is deleted in at least one open reading frame coding for the viral proteins Vif, Vpx, Vpr, Vpu, Nef, Rev, Tat, Pol, and/or Env or parts thereof. Further, the invention comprises a pharmaceutical composition comprising a VLP and method for the immunization of a patient against an infection mediated by an immunodeficiency virus.

## Description

### Field of the invention

The present invention relates to the technical field of immunology and specially to the preparation of a vaccine comprising virus-like particles (VLPs). In particular, the present invention relates to a composition for the immunization against an immunodeficiency virus, in particular HIV. The VLPs are replication defective, non-infectious and immunogenic.

### Background of the invention

The etiologic agent of acquired immune deficiency syndrome (AIDS) is a human retrovirus termed human immunodeficiency virus (HIV) of which there are presently two major subgroups, HIV-1 and HIV-2. These viruses are responsible for an ever widening world-wide epidemic of immune deficiency characterized by a slow, yet progressive, degeneration of functions of the immune and central nervous system.

The earliest symptoms of HIV infection include an acute influenza-like syndrome which persists for 2 to 3 weeks. Several weeks to many months or years following infection, lymphadenopathy and/or progressive depletion in CD4 positive T-helper lymphocytes becomes apparent and disease evolves to the point where immune deficiency becomes manifest. The diagnosis of HIV infection is confirmed by laboratory tests which include the detection of HIV-specific antibodies and/or HIV antigens in patient sera, and the isolation of infectious virus from patient body fluids or cells. A similar disease is observed in rhesus macaques infected with the simian immunodeficiency virus (SIV).

The predominant host cells for HIV in infected individuals are the CD4 positive T-helper cell and the cells of the monocyte/macrophage lineage. However, increasing evidence points to the fact that HIV can infect a wide variety of cell types, both *in vivo* and *in vitro.*

This wide host cell tropism most likely accounts for the plethora of symptoms and the severity of disease associated with HIV infection.

HIV-1 and 2 have been the subject of massive and unprecedented research efforts in recent years in a number of areas including vaccine strategies. The development of an efficacious vaccine for prevention of HIV infection is of considerable importance as it can be easily recognized that prevention of infection is the best way to combat any infectious disease.

Various strategies are currently being used in attempts to develop an effective vaccine against AIDS. Such strategies to develop a safe and efficacious AIDS vaccine include live attenuated viruses, whole inactivated viruses, subunit vaccines, recombinant viruses, genetically engineered virus-like particles, synthetic peptides, antiidiotypic antibodies, and naked plasmid DNA encoding viral proteins.

Due to the lack of an appropriate animal model, preclinical vaccine efficacy is frequently determined by immunizing non-human primates and challenge of immunized animals with SIV, which is closely related to HIV. Although live attenuated immunodeficiency viruses were shown to be effective vaccines in the monkey model (reviewed in Desrosiers, HIV Adv. Res. Ther. 3 (1996), 3-9; Johnson, Nat. Med. 5 (1999), 154-155), development of AIDS by the attenuated immunodeficiency viruses cannot be excluded (Baba et al., Science 267 (1995), 1820-1825; Baba et al., Nat. Med. 5 (1999), 194-203).

The efficacy of whole inactivated virus particles and VLPs has also been assessed. However, no relevant level of protection was observed in the monkey model if the challenge virus was produced in monkey cells, which was demonstrated by various scientific groups (Daniel et al., AIDS Res. Hum. Retroviruses 10 (1994), 839-851; Norley et al., Intervirology 45 (2002), 267-274; Lifson et al., J. Med. Primatol. 31 (2002), 205-216; Lu et al., AIDS 12 (1998), 1-10; Goldstein et al., J. Med. Primatol. 23 (1994), 75-82; Putkonen et al., J. Med. Primatol 22 (1993), 100-103; Le Grand et al., Vaccine 10 (1992), 873-879; Chan et al., J. Exp. Med. 176 (1992), 1203-1207; Stott, Nature 353 (1991), 393).

Similarly, vaccination with subunit vaccines did not lead to significant protection (Giavedoni et al., J. Virol. 67 (1993), 577-583; Stott et al., J. Gen. Virol. 79 (1998), 423-432), although sterilizing immunity could be observed early after vaccination in challenge experiments with reduced stringency (Hu et al., J. Med. Primatol. 21 (1992), 119-125; Hu et al., Science 255 (1992), 456-459; Mooij et al., AIDS 12 (1998), F15-F22).

DNA vaccines and viral vector vaccines expressing one or more immunodeficiency virus genes were able to induce antiviral immune responses and provided some level of protection in challenge experiments with reduced stringency (Shiver et al., Nature 415 (2002), 331-335; Habel et al., Dev. Biol. (2000), 101-105; Barouch et al., Immunol. Lett. 79 (2001), 57-61; Amara et al., Science 292 (2001), 69-74), while protection against neutralization resistant SIVmac239 was poor (Wang et al., J. Virol. 74 (2000), 10514-10522; Horton et al., J. Virol. 76 (2002), 7187-7202).

These approaches rely on the transfer of viral genes into cells of the vaccinee, which raises a number of safety issues. A specific form of viral vector vaccines are single cycle immunodeficiency viruses (SCIVs) developed and studied by Kuate et al., Virology 313 (2003), 653-662. In this particular case, immunodeficiency viruses were used as a vector system to transfer immunodeficiency virus genes for vaccination experiments. In contrast to live attenuated viruses, the SCIVs replicate only once in the vaccinee. After injection of SCIVs into the vaccinee, they enter cells of the vaccinee and the attenuated immunodeficiency virus genome is reverse-transcribed and integrated into the genome of the infected cells. The cells start to express immunodeficiency virus genes leading to the induction of antiviral immune response. To enhance the number of cells expressing immunodeficiency virus genes, the SCIVs can also carry the surface G-protein of the vesicular stomatitis virus (VSV-G), which allows infection of a broad spectrum of target cells.

US Patent 6,500,623 B1 also describes a replication-defective HIV particle, which is pseudo typed with VSV-G. In this particle, the pol gene of the retroviral genome is modified to cause the protease and reverse transcriptase functions of the pol genes to loose their viral replication-associated functionality. This pseudo typed HIV particle can infect many cell types, including human and simian cells. The particle also undergoes only one round of replication and may therefore be regarded as a SCIV.

Since SCIV vaccines integrate into the genome of infected cells, such approach harbors the inherent risk of insertional mutagenesis, which can lead to fatal results. The authors Hacein-Bey-Abina and co-workers have recently reported about a correction of X-linked severe combined immunodeficiency (SCID-X1, also known as gamma chain (gamma(c)) deficiency) in 9 out of 10 patients by retrovirus-mediated gamma(c) gene transfer into autologous CD34 bone marrow cells. However, almost 3 years after gene therapy, uncontrolled exponential clonal proliferation of mature T-cells (with gammadelta+ or alphabeta+ T cell receptors) has occurred in the two youngest patients. Both patients' clones showed retrovirus vector integration in the proximity to the LMO2 proto-oncogene promoter, leading to aberrant transcription and expression of LMO2. Thus, it is now clear that retrovirus vector insertion can trigger deregulated premalignant cell proliferation with unexpected frequency, most likely driven by retrovirus enhancer activity on the LMO2 gene promoter (Hacein-Bey-Abina et al., Science 302 (2003), 415-419).

It is evident from the above mentioned problems that there is a strong need for a composition for immunization against an immunodeficiency virus, which does not lead to the transfer of viral genes and thus avoids insertional mutagenesis on all accounts. However, such vaccine should nevertheless provide strong protection against infection by an immunodeficiency virus.

The solution to this technical problem is achieved by providing the embodiments characterized in the claims, and described further below.

### Summary of the invention

The present invention is directed to use of a virus-like particle (VLP) for the preparation of a composition for immunization against an immunodeficiency virus, wherein said VLP is formed by at least a part of the viral protein Gag, said VLP comprises an envelope with at least one viral protein or a part thereof and at least one heterologous protein, said VLP is replication defective, and the at least one nucleic acid coding for said VLP is deleted in at least one open reading frame coding for the viral proteins Vif, Vpx, Vpr, Vpu, Nef, Rev, Tat, Pol, and/or Env or parts thereof.

It is another object of the present invention to provide a pharmaceutical composition comprising a virus-like particle (VLP) for immunization against an immunodeficiency virus, wherein said VLP is formed by at least a part of the viral protein Gag, said VLP comprises an envelope with at least one viral protein or a part thereof and at least one heterologous protein, said VLP is replication defective, and the at least one nucleic acid coding for said VLP is deleted in at least one open reading frame coding for the viral proteins Vif, Vpx, Vpr, Vpu, Nef, Rev, Tat, Pol, and/or Env or parts thereof.

The present invention also concerns a method for the immunization of a patient against an infection mediated by an immunodeficiency virus, comprising the step of administering to a patient an effective amount of a pharmaceutical composition comprising a virus-like particle (VLP) for immunization against an immunodeficiency virus, wherein said VLP is formed by at least a part of the viral protein Gag, said VLP comprises an envelope with at least one viral protein or a part thereof and at least one heterologous protein, said VLP is replication defective, and the at least one nucleic acid coding for said VLP is deleted in at least one open reading frame coding for the viral proteins Vif, Vpx, Vpr, Vpu, Nef, Rev, Tat, Pol, and/or Env or parts thereof.

In a preferred embodiment, the immunodeficiency virus may be HIV, in particular HIV-1 or HIV-2.

In order to ensure the replication deficiency to avoid insertional mutagenesis by integration, the nucleic acid coding for the VLP is mutated in the primer binding site.

In a particular preferred embodiment, the heterologous protein is the vesicular stomatitis virus G-protein (VSV-G). Alternatively, the heterologous protein can be derived from an enveloped virus other than VSV, or it can be an antibody, in particular a single chain antibody. Further, the heterologous protein may be a ligand or modified ligand of a specific human cell surface receptor.

### Brief description of the drawings

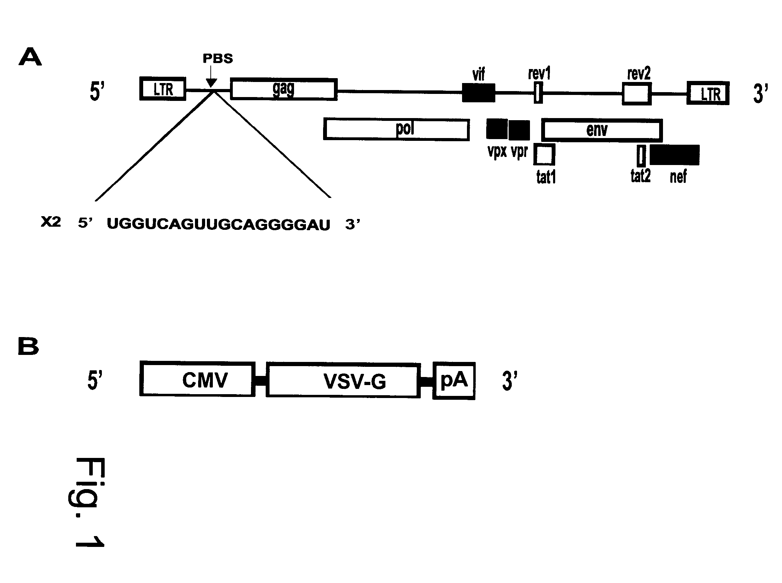

**Fig. 1** shows a map of an expression plasmid for the VLP, designated SX2Δfrxn (A) and an expression plasmid for the G-protein of the vesicular stomatitis virus (VSV-G), termed pHIT-G (B). In panel (A), the open reading frames and relevant regulatory elements of the simian immunodeficiency virus (SIV) are indicated by white boxes, inactivated reading frames are indicated in dark. The sequence of the mutated primer binding site (PBS), designated X2, is shown. In panel (B) the term "CMV" indicates the enhancer, promoter and first intron of the human cytomegalovirus immediate early gene, and the term "VSV-G" indicates the gene encoding the G-protein of the vesicular stomatitis virus. The term "pAd" indicates the polyadenylation signal of SV40.

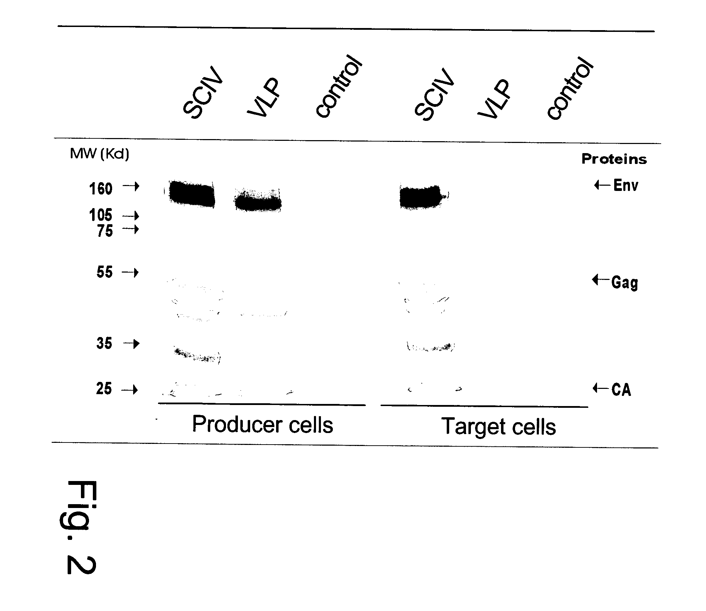

**Fig. 2** shows a Western Blot analysis of SIV virus-like particles (VLPs) in transfected and infected cells. To generate VSV-G containing VLPs, 293T producer cells were transfected with SX2Δfrxn and pHIT-G. The supernatant of transfected producer cells was incubated with 293A target cells. To detect VLPs, the supernatants of the producer cells and target cells were pelleted through a 20 % sucrose cushion and the pellets were analyzed by Western Blot with a serum from an SIV-infected monkey. For comparative reasons single cycle immunodeficiency viruses (SCIV) were also employed. Particle formation of SCIV was also analyzed in transfected producer cells and infected target cells.

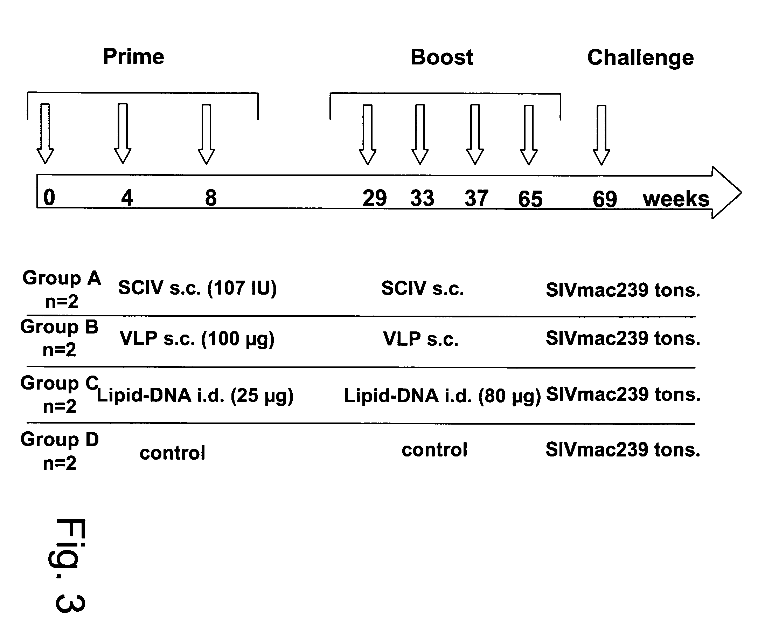

**Fig. 3** shows the outline of the immunization schedule. 4 groups with 2 monkeys were each immunized and challenged at the indicated weeks after the first injection. "s.c." stands for subcutaneous injection, "i.d." stands for intradermal injection, "tons" stands for tonsillar challenge. Tonsillar challenge has been described by Stahl-Hennig (Stahl-Hennig et al., Science 285 (1999), 1261-1265).

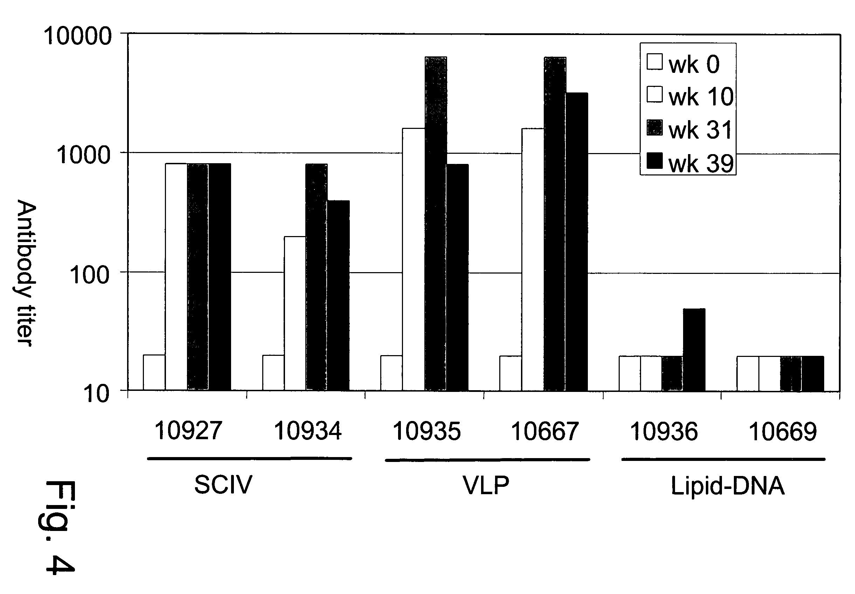

**Fig. 4** shows antibody titers to SIV capsid protein, and the reciprocal of the antibody titers at the indicated weeks (wk) after the first immunization are given. The 5 digit numbers under the bars are monkey designations, and the compositions used are given below the monkey designations.

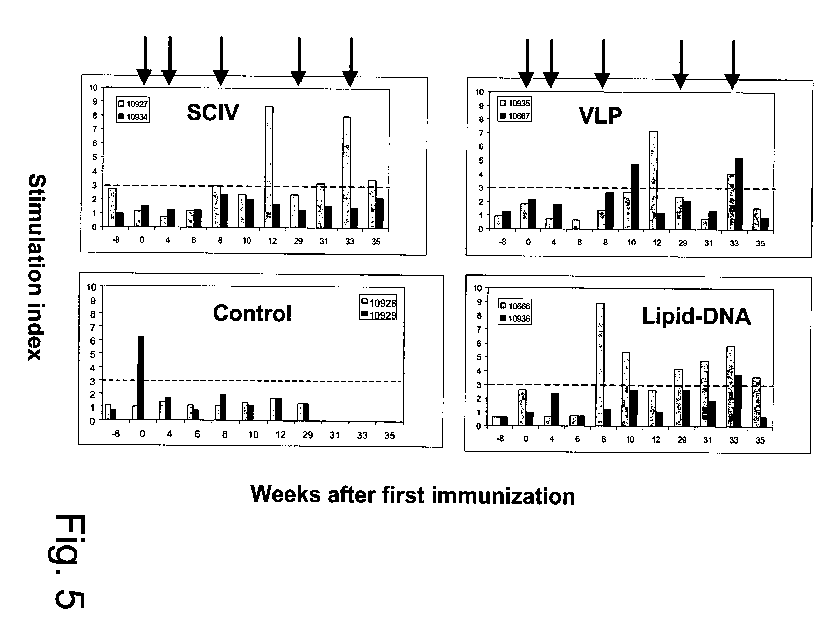

**Fig. 5** shows the lymphoproliferative response to the SIV Gag protein. Peripheral blood mononuclear cells (PBMCs) from immunized monkeys at the indicated weeks after the first immunization were stimulated with SIV Gag-VLP and proliferation was determined by ³[H]-thymidine incorporation. Arrows mark the time points of immunization. The stimulation index was calculated by dividing the mean count of the antigen-containing cultures by that of cultures lacking the antigen. Stimulation indices >3 were considered positive. Animal designations are as in Fig 4.

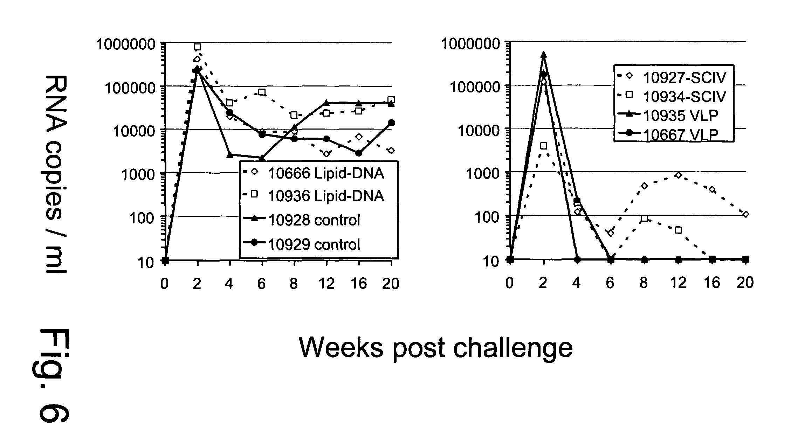

**Fig. 6** shows viral RNA levels after challenge. Monkeys were exposed to a tonsillar challenge with approximately 5000 TCID₅₀ (50 % of the tissue culture infectious dose) of SIVmac239 4 weeks after the last immunization. Viral RNA levels in the plasma at the indicated weeks after challenge are given. 5 digit numbers in the figure legends are animal designations and are followed by the vaccine composition used in the respective monkey.

### Detailed description of the invention

The present invention relates to the use of a virus-like particle (VLP) for the preparation of a composition for immunization against an immunodeficiency virus, wherein said VLP is formed by at least a part of the viral protein Gag, said VLP comprises an envelope with at least one viral protein or a part thereof and at least one heterologous protein, said VLP is replication defective, and the at least one nucleic acid coding for said VLP is deleted in at least one open reading frame coding for the viral proteins Vif, Vpx, Vpr, Vpu, Nef, Rev, Tat, Pol, and/or Env or parts thereof.

Current theory in vaccine development against infection mediated by immunodeficiency viruses predicts that transfer of lentiviral genes and expression of lentiviral genes by cells of the vaccinee is important for good immunogenicity and vaccine protection. This theory is based on experiments showing that vaccination with non-infectious lentiviral particles does not provide protection in animal models against AIDS. The inventors of the present invention have surprisingly found out that non-infectious lentiviral particles modified by incorporation of a heterologous protein induce good immune responses and protection *in vivo.*

The present invention describes the use of an effective vaccine against immunodeficiency viruses. The incorporation of appropriate heterologous surface proteins into immunodeficiency virus-like particles (VLPs) leads to good immunogenicity and vaccine efficacy. While not wanting to be bound by a particular theory, the heterologous proteins are presumed to enhance vaccine efficacy by increasing binding, uptake, intracellular processing and/or presentation of the VLPs by antigen presenting cells.

In the prior art, infectious vesicular stomatitis virus G-protein (VSV-G) pseudo typed lentiviral particles have been used to transfer lentiviral RNA in a broad spectrum of target cells for gene transfer (Naldini et al., Science 272 (1996), 263-267) and vaccination experiments, leading to the integration of the proviral DNA generated by reverse transcription of the lentiviral RNA into the genome of target cells. These vectors harbor the risk of insertional mutagenesis. Therefore, VLPs carrying a heterologous protein were generated that lack a lentiviral RNA that can be reverse transcribed. These particles are non-infectious and cannot even undergo a single cycle of replication.

It is a particular advantage that the VLP of the present invention is non-infectious and absolutely replication deficient both in cell culture and *in vivo* in vaccinated animal or human subjects, so that it entirely avoids the risks associated with the insertion of the proviral DNA into the host's genome, which is known by the term "insertional mutagenesis". This safety aspect is efficiently combined with a good immune response and protection against viral challenge. The present invention avoids an intact viral genome, which is also particularly advantageously with respect to safety issues in human vaccinees. The nucleic acid coding for the VLP is deleted in the coding sequence of a least one viral protein. Additionally, the invention encompasses VLPs, which are encoded by more than one nucleic acid molecule. For example, the coding sequence for the VLP may be encoded by different plasmids, each comprising a part of the coding sequence of the VLP.

Therefore, the present invention provides an efficient vaccine, which does not lead to the transfer of viral genes, but nevertheless provides strong protection against viral infection.

### Definitions

In describing the present invention, the following terms will be employed, and are intended to be defined as indicated below.

As used herein, the term "virus-like particle" or "VLP" refers to a non-replicating, viral shell, derived from any of several viruses discussed further below. VLPs are generally composed of one or more viral proteins, such as, but not limited to those proteins referred to as capsid, coat, shell, surface and/or envelope proteins, or particle-forming polypeptides derived from these proteins. VLPs can form spontaneously upon recombinant expression of the protein in an appropriate expression system. Methods for producing particular VLPs are known in the art and discussed more fully below. The presence of VLPs following recombinant expression of viral proteins can be detected using conventional techniques known in the art, such as by electron microscopy, biophysical characterization, and the like. See, e.g., Baker et al., Biophys. J. 60 (1991), 1445-1456; Hagensee et al., J. Virol. 68 (1994), 4503-4505. For example, VLPs can be isolated by density gradient centrifugation and/or identified by characteristic density banding. Alternatively, VLPs can be derived from the supernatant of transfected cells (e.g. Example 1).

The term "immunization" means an immunological response to an antigen or composition, developed in a subject. "Immunization" means a humoral and/or a cellular immune response to an antigen present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells (CTLs). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, non-specific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art.

Thus, an "immunization" or immunological response as used herein may be one which stimulates the production of CTLs and/or the production or activation of helper T-cells. The antigen of interest may also elicit an antibody-mediated immune response. Hence, an immunological response may include one or more of the following effects: the production of antibodies by B-cells; and/or the activation of suppressor T-cells and/or gammadelta T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

For an "immunization" with a VLP according to the present invention the composition is administered to a subject and results in the development of a humoral and/or a cellular immune response to the antigenic molecule of interest in the subject.

A "coding nucleic acid" or a nucleic acid which "encodes" a selected protein, is a nucleic acid molecule which is transcribed and translated into a protein *in vivo* when placed under the control of appropriate regulatory sequences or "control elements". The boundaries of the coding sequence are determined by a start codon at the 5' (amino) terminus and a translation stop codon at the 3' (carboxy) terminus.

Typical "control elements", include, but are not limited to, transcription promoters, transcription enhancer elements, transcription termination signals, polyadenylation sequences (located 3' to the translation stop codon), sequences for optimization of initiation of translation (located 5' to the coding sequence), and translation termination sequences, see e.g., McCaughan et al., PNAS USA 92 (1995), 5431-5435; Kochetov et al., FEBS Letts. 440 (1998), 351-355.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

As used herein, the term "treatment" refers to any of (i) the prevention of infection or reinfection, as in a traditional vaccine, (ii) the reduction or elimination of symptoms, and (iii) the substantial or complete elimination of the pathogen in question. Treatment may be effected prophylactically (prior to infection) or therapeutically (following infection).

As used herein, the term "heterologous" in connection with "heterologous protein" means a protein which does not naturally occur in an immunodeficiency virus. Examples for a heterologous protein is the G-protein of the vesicular stomatitis virus (VSV-G), any other viral proteins not naturally occurring in immunodeficiency viruses, like antibodies, ligands and the like.

As used in the context of the present invention, the term "replication defective" means, that the VLP is not even able to undergo a single cycle of replication. The "replication defective" VLP of the present invention does not encode a nucleic acid which integrates in the host's genome. Thus, the VLP of the present invention does not lead to insertional mutagenesis.

### Modes of carrying out the invention

In general terms, the replication cycle of lentiviruses, including HIV-1, closely resembles that of other retroviruses. There are, however, a number of unique aspects of HIV replication; for example, the HIVs and SIVs target receptors and coreceptors distinct from those used by other retroviruses. Lentiviruses encode a number of regulatory and accessory proteins not encoded by the genomes of the prototypical "simple" retroviruses. Of particular interest from the gene therapy perspective, lentiviruses possess the ability to productively infect some types of non-dividing cells. The HIV-1 genome encodes the major structural and non-structural proteins common to all replication-competent retroviruses. From the 5'- to 3'-ends of the genome are found the gag (for group-specific antigen), pol (for polymerase), and env (for envelope glycoprotein) genes. The gag gene encodes a polyprotein precursor whose name, Pr55Gag, is based on its molecular weight. Pr55Gag is cleaved by the viral protease (PR) to the mature Gag proteins matrix (also known as MA or p17), capsid (CA or p24), nucleocapsid (NC or p7), and p6. Two spacer peptides, p2 and p1, are also generated upon Pr55Gag processing. The pol-encoded enzymes are initially synthesized as part of a large polyprotein precursor, Pr160GagPol, whose synthesis results from a rare frameshifting event during Pr55Gag translation. The individual pol-encoded enzymes, PR, reverse transcriptase (RT), and integrase (IN), are cleaved from Pr160GagPol by the viral PR. The envelope (Env) glycoproteins are also synthesized as a polyprotein precursor. Unlike the Gag and Pol precursors, which are cleaved by the viral PR, the Env precursor, known as gp160, is processed by a cellular protease during Env trafficking to the cell surface, gp160 processing results in the generation of the surface (SU) Env glycoprotein gp120 and the transmembrane (TM) glycoprotein gp41. gp 120 contains the determinants that interact with receptor and coreceptor, while gp41 not only anchors the gp120/gp41 complex in the membrane, but also contains domains that are critical for catalyzing the membrane fusion reaction between viral and host lipid bilayers during virus entry. Comparison of env sequences from a large number of virus isolates revealed that gp120 is organized into five conserved regions (C1-C5) and five highly variable domains (V1-V5). The variable regions tend to be located in disulfide-linked loops. gp41 is composed of three major domains: the ectodomain (which contains determinants essential for membrane fusion), the transmembrane anchor sequence, and the cytoplasmic tail. In addition to the gag, pol, and env genes, HIV-1 also encodes a number of regulatory and accessory proteins. Tat is critical for transcription from the HIV-1 long terminal repeat and Rev plays a major role in the transport of viral RNAs from the nucleus to the cytoplasm. Vpu, Vif, Vpr and Nef have been termed "accessory" or "auxiliary" proteins to reflect the fact that they are not uniformly required for virus replication. These very interesting proteins play an important role in this invention, as they may be mutated in the VLP according to the present invention. HIV replication proceeds in a series of events that can be divided into two overall phases: "early" and "late". Although some events occur in a concerted or simultaneous fashion, the replication cycle can be viewed most simply as proceeding in an ordered, step-wise manner.

One aspect of the present invention is the generation of a virus-like particle (VLP) for the preparation of a composition for immunization against an immunodeficiency virus. The immunodeficiency virus may be the human immunodeficiency virus (HIV), in particular HIV-1 or HIV-2. The VLP is formed by at least a part of the viral protein Gag. The Gag protein wild-type sequences can be obtained from the HIV-1SF2 strain (Sanchez-Pescador et al., Science 227 (4686) (1985), 484-492; US 5,156,949; US 5,688,688). Gag sequence obtained from other HIV variants may also be used. Such other variants include, but are not limited to Gag protein encoding sequences obtained from the isolates HIV.sub.IIIb, HIV.sub.SF2, HIV-1.sub.SF162, HIV-1.sub.SF170, HIV.sub.LAV, HIV.sub.LAI, HIV.sub.MN, HIV-1.sub.CM235, HIV-1.sub.US4, other HIV-1 strains from diverse subtypes (e.g., subtypes, A through G, and O), HIV-2 strains and diverse subtypes (e.g., HN-2.sub.UC1 and HIV-2.sub.UC2), and feline and simian immunodeficiency virus. (See, e.g., Fundamental Virology, 2nd Edition (Fields and Knipe, eds. 1991); Virology, 3rd Edition (Fields, Knipe, Howley, eds. 1996, Lippincott-Raven, Philadelphia, PA; for a description of these and other related viruses).

It is also encompassed by the present invention that a fusion protein comprising at least a part of an immunodeficiency virus Gag protein, in particular the HIV Gag protein and a heterologous protein can be used. If such a fusion protein is used for vaccination of a subject, the inventors presume, without being bound by any theory, that the part of the fusion protein derived from the heterologous protein targets a specific cell surface receptor leading to binding, uptake, intracellular processing and/or presentation of the entire fusion protein. These steps may be the same or at least highly similar to the uptake of enveloped viruses, like HIV, influenza viruses or the like. Also VLPs according to the present invention may enter cells via this route.

The fusion protein has the advantage that it is expressed from one nucleic acid, leading to an equal amount of the HIV protein and the heterologous protein. A fusion protein can be conveniently expressed in *Escherichia coli,* it can be produced in large amounts and is easy to purify. Native proteins can be produced in bacteria by placing a strong, regulated promoter and an efficient ribosome-binding site upstream of the cloned gene.

After binding of the fusion protein or the VLP to the cell surface a coated pit is formed. An endocytotic vesicle then pinches off with the fusion protein or the VLP inside. Acidification of the interior of the vesicle, called an endosome, is then promoted by a proton pump in the membrane. As the pH drops, at least one VLP or virion surface protein undergoes a conformational change, causing exposure of a hydrophobic portion of the VLP or virion surface protein. This hydrophobic region promotes fusion between the VLP or virion lipid envelope and the vesicle membrane, releasing the nucleocapsid into the cytoplasm. At this point, the viral genetic material has entered the host cell. Thus, for enveloped viruses, this mechanism is similar to surface fusion, expect that fusion of the viral envelope with the cell membrane occurs within the host cell. The main differences appear to be trigger mechanisms activating fusion and thus the cellular sites, at which fusion occurs.

Entry of the cell via surface fusion is also encompassed. The fusion protein or the VLP fuses with the cell plasma membrane at the cell surface. Binding of the VLP to the cell-surface receptor leads to fusion between the VLP lipid envelope and the cell plasma membrane. This releases the viral nucleocapsid into the cytoplasm of the host-cell plasma membrane. The surface fusion of enveloped VLPs is promoted by VLP surface proteins. A series of events is believed to lead to fusion of the envelope with the cell plasma membrane, which was studied in detail for paramyxoviruses.

The minimal part of the viral Gag protein, which is still considered to be able to form a VLP, is the N-terminal part of Gag containing a membrane anchoring motif and the major homology region of the capsid protein. If such a minimal VLP is used, the N-terminus of the matrix protein is associated with the membrane. Even though there is no extracellular part of the protein, the N-terminus of the matrix protein is at least membrane associated. In a particularly preferred embodiment, the Gag protein carries a myristic acid residue and is therefore myristoylated.

The VLP comprises an envelope with at least one viral protein or a part thereof. The viral protein forming said VLP is selected from the group consisting of Gag, Pol, Env, Tat, Rev, Nef, Vif, Vpr, Vpx and Vpu. In a preferred embodiment the Pol protein comprises a reverse transcriptase (RT), an integrase (IN) and/or a protease (PR).

Infectious virus of wild-typ HIV and SIV contain two identical copies of single-stranded RNA, about 9.2 kb long, that have positive polarity with respect to translation. In the early stages of infection, the virion RNA genome is converted into double-stranded linear DNA by the process of reverse transcription, via viral-encoded reverse transcription (RT), which involves two strand-transfer steps to synthesize linear viral DNA with long terminal repeats (LTRs) flanking viral genes. This linear viral DNA is integrated into the host cell genome to produce the provirus. Accordingly, HIV and SIV, like other retroviruses, have two genomic forms: single-stranded RNA in the extracellular phase of the viral life cycle (i.e., virions) and double-stranded DNA (i.e., provirus) within the cell. Genomic viral DNA and thus contains a cap structure at the 5' end a poly-A tail at the 3' end.

Models for the structure of HIV and SIV virions are based on a combination of high-resolution electron microscopy of viral particles and both biochemical and immunochemical analyses of virion components. By these approaches, extracellular particles produced by cells infected with HIV-1, HIV-2, and the various SIV strains are very similar in morphology and composition. Therefore, SIV serves as animal model for HIV since the early days of research in the field. Virions have spherical shape, are about 110 nm in diameter, and consist of a lipid bilayer membrane or envelope that surrounds the cone-shaped nucleocapsid.

Biochemical and immunochemical analysis has demonstrated that the nucleocapsid within each mature virion is composed of two molecules of the viral single-stranded RNA genome encapsulated by proteins proteolytically processed from the Gag precursor polypeptide. These gag gene products are the matrix protein (MA), which is presumably located between the nucleocapsid and the virion envelope (membrane); the major capsid protein (CA), which forms the capsid shell (5 nm thick); and the nucleocapsid protein (NC), which binds tightly to the viral RNA genome. The 5' ends of the two molecules of genomic single-stranded viral RNA (∼ 9.2 kb) in the virion appear to interact with each other either through hydrogen bonds between molecules aligned in the same polarity or through short anti-parallel arrangements; NC protein may also link the two viral genomes. A transfer RNA^{lys} molecule is positioned near the 5' end of each genomic RNA strand and serves as the primer of initiation of negative strand viral DNA synthesis by RT. A very small proportion of extracellular virus particles also contains viral DNA produced by incomplete reverse transcription which takes place after release of virions from cells. Viral enzymes derived from the pol gene precursor polypeptide are also packaged into virions; these enzymes are PR, RT, and IN. Reverse transcriptase is a heterodimer composed of p51 ad p66 subunits. An additional enzymatic activity, ribonuclease specific to RNA in RNA-DNA hybrids (RNase H), is an independent domain in the RT heterodimer. Reverse transcriptase (and RNase H) and IN are presumably contained with nucleocapsids; the precise relationship of these two accessory proteins with other capsid proteins is not well defined.

The membrane (or envelope) of extracellular HIV and SIV particles contains approximately 72 knobs (or spikes) that show triangular symmetry. Each knob is 9 to 10 nm long and has an ovoid distal end, 14 to 15 nm in diameter, linked to the lipid membrane by a 7-to-8-nm stalk. The knob is thought to contain 3 heterodimers of the Env gp, and each heterodimer is composed of a surface (SU) subunit that interacts with the transmembrane (TM) subunit through noncovalent bonds. Human immunodeficiency virus type 1 SU and TM subunits are designated gp120 and gp41, respectively, on the basis of electrophoretic mobility under denaturing conditions in polyacrylamide gels. The counterpart subunits for HIV-2 and SIV Env gp are gp130 and gp41. The SU of the primate lentiviruses is extensively glycosylated ∼50 kd of protein backbone and ∼70 kd of carbohydrate side groups and contains a domain that recognizes and binds the cell receptor; this receptor for HIV and SIV is the CD4 antigen, a cell surface protein on T-helper lymphocytes.

In a preferred embodiment of the present invention, the viral protein forming the VLP is mutated or modified to increase immunogenicity and/or representation of the antigenic variant of HIV.

For example, env-based vaccine immunogens are encompassed in which the conserved functional domains of gp120 are better exposed. It was shown that the presence of a highly conserved N-linked glycosylation site located at the N-terminus of the V3 loop modifies the structure of the V3 loop and obstructs access to the highly conserved CD4 binding and CD4i sites of gp120 from diverse HIV-1 isolates (Malenbaum et al., J. Virol. 74 (2000), 11008-11016). The use of V3 loop deglycosylated Envs as vaccine components may therefore elicit broadly cross-reactive neutralizing activity. Moreover, by assessing the degree to which the V3 loop glycan affects susceptibility of the virus to neutralization by sera from infected humans and macaques, one may gain insights into the similarities or differences in antigenic recognition of the HIV-1 envelopes by humans and macaques. Furthermore, the extent to which the conserved neutralization epitopes of gp120 are immunogenic in the two hosts could be evaluated. The combinations of SIV and HIV, termed "SHIVs" carry the envelope gene of the T-cell-line-tropic, CXCR4-using (X4), and V3 loop glycan-deficient strain HIV-1_{SF33} or the macrophage-tropic, CCR5-using (R5), and V3 glycan-possessing isolate HIV-1_{SF162} (SHIV_{SF33} and SHIV_{SF162}, respectively) (Luciw et al., PNAS USA 92 (1995), 7490-7494). Immunization with glycan-deficient envelopes better expose highly conserved epitopes and elicit more potent broadly cross-neutralization antibodies.

According to another preferred embodiment, the VLP according to the present invention is formed by two or more of the viral proteins with Vif, Vpx, Vpr, Vpu, Nef, Ref, Tat, Pol and/or Env or parts thereof.

Further, the VLP according to the present invention is replication defective. The replication deficiency is mediated by a mutation in the primer binding site of the immunodeficiency virus. The primer binding site of SIV or HIV can be mutated to a sequence designated X2, as indicated in Fig. 1. This mutation avoids complementary to known cellular tRNAs (Lund et al., J. Virol. 71 (1997), 1191-1195). This mutation results in a defect in reverse transcription which leads to a replication defective VLP.

In a preferred embodiment of the present invention, the vif gene was also deleted. Vif, which is required for virus replication in primary cells (Gabuzda et al., J. Virol. 66 (1992), 6489-6495), counteracts a cellular factor inhibiting production of infectious virus particles (Sheehey et al., Nature 418 (2002), 646-650). In some cell lines, such as 293T cells, the antiviral factor is not expressed, allowing the production of infectious viral particles in the absence of vif. Infection of primary cells with these vif-deficient SCIVs leads to production of SIV particles that are noninfectious due to the vif deficiency. In addition to this approach, the accessory genes vpr, vpx and nef were also deleted to further attenuate the VLP, to prevent down regulation of MHC-I by Nef (Piguet et al., Immunol. Rev. 168 (1999), 51-63), and to avoid Vpr-induced cell cycle arrest (Planelles et al., J. Virol. 70 (1996), 2516-2524).

According to another aspect of the present invention, the VLP is integration defective, which means that the VLP does not integrate in the host's genome.

Further, the envelope of the VLP is a lipid membrane surrounding the VLP. This lipid membrane is the derived from a host, which may be a enkaryotic cell. The host is selected from insect cells, yeast cells and/or mammalian cells. Among insect cells, the cells of Spodoptera frugiperda (baculovirus system) are preferred. As far as yeast cells are concerned, cells of *Saccharomyces cerevisiae* or *Schizosaccharomyces pombe* are preferred. Among mammalian cells, in particular human cell lines, like HeLa cells and 293 cells or primary human cells are preferred. Further, monkey cells, in particular monkey cell lines like COS cells or VERO cells may be used.

In another embodiment of the present invention, the VLP may be formed *in vitro.* An E. coli expressed Gag protein may be combined with a heterologous protein, which may also be expressed in E. coli. An envelope may comprise lipids which are synthetically manufactured or derived from a natural source.

In a preferred embodiment, HIV particles containing gag, gag-pol, and env of HIV-1 or HIV-2 and the G-protein of VSV are used for vaccination. Therefore, the heterologous protein is the vesicular stomatitis virus G-protein (VSV-G). However, other combinations of HIV proteins can also be used. Incorporation of VSV-G into HIV-Iike particles allows attachment of the particles to a wide spectrum of human and animal cells. After endocytosis of the HIV-like particles, a reduction in the pH leads to VSV-G-mediated fusion of the membrane of the HIV-like particles with the membrane of the endosome resulting in cytoplasmic delivery of HIV proteins. In contrast to single cycle immunodeficiency virus (SCIV) vaccines, the lentiviral genome of the HIV-like particle is not reverse transcribed and/or integrated into the genome of the target cells, since in the preferred embodiment packaging and/or reverse transcription of the lentiviral genome is blocked. Therefore, immunodeficiency virus genes are not expressed in the vaccinees. Only the HIV proteins being part of the particles can induce antiviral immune responses.

Since the VSV-G protein is also part of the particle an immune response to VSV-G is also generated. Other viral surface proteins such as the envelope protein of murine leukemia virus might therefore be beneficial for booster immunization and could also be used.

Instead of a viral surface protein, an antibody including a single chain antibody targeting a specific cell surface receptor can also be used to increase binding, uptake, intracellular processing and/or presentation of the immunodeficiency virus-like particles by antigen presenting cells. Alternatively, a natural ligand of a cell surface receptor can be engineered to be incorporated into the membrane of HIV-like particles. It is evident, that combinations of viral surface proteins, targeting antibodies and or ligands could also be used. It is also encompassed that combinations of VSV-G, antibodies and/or other viral surface proteins can be used.

The examples provided below use transient cotransfection of mammalian cells with expression plasmids encoding lentiviral genes and VSV-G for the production of immunodeficiency virus-like particles carrying VSV-G. However, other production systems could also be used. Cell lines could be established that stably or after induction produce HIV-like particles containing VSV-G. Viral vector systems (such as adenoviral vectors) encoding the HIV-proteins and VSV-G could also be used to generate mammalian cells producing the HIV-like particles containing VSV-G. Production is not limited to mammalian cells. Insect cells and yeast cells could also be engineered to produce the HIV-like particles containing VSV-G. In vitro assembly of HIV-like particles containing VSV-G or other heterologous surface proteins from prokaryotically expressed immunodeficiency virus proteins and heterologous surface proteins and synthetic lipids can also be envisioned.

In yet another preferred embodiment, the heterologous protein is the vesicular stomatitis virus G-protein (VSV-G), which can be mutated or modified. The mutation or modification may be employed in any amino acid residue which enhances or augments the induction of a humoral or cellular immune response. Additionally, the heterologous protein may also be derived from an enveloped virus other than VSV. For example, other viral surface proteins, such as the envelope protein of murine leukemia virus can be used. Further, the heterologous protein can be an antibody including single-chain antibodies targeting a specific cell surface receptor. The cell surface receptor may be of human origin.

Moreover, the heterologous protein may be a ligand or modified ligand of a cell surface receptor. Suitable receptors are for example the granulocyte monocyte colony stimulating factor (GM-CSF) receptors and toll-like receptors. In a particular preferred embodiment the cell surface receptor is of human origin.

It is also encompassed that two or more different or equal heterologous proteins are used. For example, the VSV G-protein may be combined with an antibody, especially single-chain antibodies targeting specific cell surface receptors.

In a preferred embodiment, the VLP of the present invention is used for immunization of a subject and, preferably the subject is human. The immunization may be a prophylactic or therapeutic immunization, which means, that the treatment of an HIV infection in a human is either prophylactic or curative.

The composition comprising the VLP further comprises a pharmaceutically acceptable carrier. Further, a pharmaceutically suitable excipient may also be comprised, which includes, among others, cytokines and adjuvants.

The vaccine based on VLPs may further comprise suitable, i.e., physiologically acceptable, carriers preferably for the preparation of injection solutions and further additives as usually applied in the art (stabilizers, preservatives, etc.), as well as additional drugs. The patients may be administered a dose of approximately 1 to 10 µg/kg body weight, preferably by intravenous injection once a day. For less threatening cases or long-lasting therapies the dose may be lowered to 0.5 to 5 µg/kg body weight per day. The treatment may be repeated in periodic intervals, e.g., 2 to 3 times per day, or in daily or weekly intervals, depending on the status of the infection.

The retrovirus-like particle preferably is one in which the env, pol and gag gene products correspond to the env, pol and gag gene products of a human retrovirus, particularly HIV-1, HIV-2, HTLV-1 or HTLV-2. Specifically, the human retrovirus may be HIV-1 and the env gene product may be an LAI env gene product, an MN env gene product, an env gene product from a primary HIV-1 isolate, or an env gene product antigenically equivalent thereto.

The present invention further includes, in an additional aspect, an immunogenic composition capable of eliciting a retroviral specific immune response and a specific immune response against a non-retroviral marker, comprising the retrovirus-like particles and a carrier therefore. Such composition may be formulated for mucosal or parenteral administration, by oral, anal, vaginal or intranasal routes. The immunogenic composition may comprise at least one other immunogenic or immunostimulating material, specifically an adjuvant, such as aluminum phosphate, aluminum hydroxide, Freund's incomplete adjuvant or QS21.

In a particularly preferred embodiment, the present invention further encompasses a pharmaceutical composition comprising a virus-like particle (VLP) for immunization against an immunodeficiency virus, wherein said VLP is formed by at least a part of the viral protein Gag, said VLP comprises an envelope with at least one viral protein or a part thereof and at least one heterologous protein, said VLP is replication defective, and the at least one nucleic acid coding for said VLP is deleted in at least one open reading frame coding for the viral proteins Vif, Vpx, Vpr, Vpu, Nef, Rev, Tat, Pol, and/or Env or parts thereof.

Vaccines may be prepared as injectables, as liquid solutions or emulsions. The non-infectious retrovirus-like particles may be mixed with pharmaceutically-acceptable excipients which are compatible with the retrovirus-like particles. Excipients may include water, saline, dextrose, glycerol, ethanol, and combinations thereof. The vaccine may further contain auxiliary substances, such as wetting or emulsifying agents, pH buffering agents, or adjuvants to enhance the effectiveness of the vaccines. Methods of achieving an adjuvant effect for the vaccine include the use of agents, such as aluminum hydroxide or phosphate (alum), commonly used as 0.05 to 0.1 percent solution in phosphate buffered saline and other adjuvants, including QS21 and incomplete Freunds adjuvant. Vaccines may be administered parenterally, by injection subcutaneously or intramuscularly. Alternatively, the immunogenic compositions formed according to the present invention, may be formulated and delivered in a manner to evoke an immune response at mucosal surfaces. Thus, the immunogenic composition may be administered to mucosal surfaces by, for example, the nasal or oral (intragastric) routes. Alternatively, other modes of administration including suppositories and oral formulations may be desirable. For suppositories, binders and carriers may include, for example, polyalkalene glycols or triglycerides. Oral formulations may include normally employed incipients, such as pharmaceutical grades of saccharine, cellulose and magnesium carbonate. These compositions take the form of solutions, suspensions, tablets, pills, capsules, sustained-release formulations or powders and contain 10 to 95 % of the VLP of the invention.

The vaccines are administered in a manner compatible with the dosage formulation, and in such amount as is therapeutically effective, protective and immunogenic. The quantity to be administered depends on the subject to be treated, including, for example, the capacity of the individual's immune system to synthesize antibodies, and to produce a cell-mediated immune response. Precise amounts of active ingredient required to be administered depend on the judgment of the practitioner. However, suitable dosage ranges are readily determinable by one skilled in the art and may be of the order of micrograms of the retrovirus-like particles. Suitable regimes for initial administration and booster doses are also variable, but may include an initial administration followed by subsequent administrations. One example of an immunization schedule is at least one pre-immunization with a VLP according to the present invention followed by at least one secondary immunization with a VLP or a synthetic peptide, as e.g. described in the published EP 0 570 980. The dosage of the vaccine may also depend on the route of administration and will also vary according to the size of the host.

VLPs in accordance with the invention may further find use in the treatment (prophylactic or curative) of AIDS and related conditions, by inducing, enhancing and/or modulating the antiviral immune responses in a subject.

A further aspect of the invention thus provides a method for the immunization of a patient against an infection mediated by an immunodeficiency virus, comprising the step of administering to a patient an effective amount of a composition according to the present invention. The patient may preferably be a person infected by HIV.

### Examples

The examples which follow further illustrate the invention, but should not be construed to limit the scope of the invention in any way. Detailed descriptions of conventional methods, such as those employed herein can be found in the cited literature; see also "The Merck Manual of Diagnosis and Therapy" 17^{th} ed. by Beers and Berkow (Merck & Co., Inc. 2003). The practice of the present invention will employ, unless otherwise indicated, conventional techniques of cell biology, cell culture, molecular biology, transgenic biology, microbiology, recombinant DNA, and immunology, which are within the skill of the art. Methods in molecular genetics, genetic engineering, oligonucleotide synthesis and DNA cloning are described generally in the current editions of Molecular Cloning: A Laboratory Manual, (Sambrook et al., (1989) Molecular Cloning: A Laboratory Manual, 2nd ed., Cold Spring Harbor Laboratory Press). Immunochemical methods are described in: Cell and Molecular Biology (Mayer and Walker, eds., Academic Press, London, 1987); Handbook of Experimental Immunology, Volumes I-IV (Weir and Blackwell, eds., 1986). Reagents, cloning vectors, and kits for genetic manipulation referred to in this disclosure are available from commercial vendors such as BioRad, Stratagene, Invitrogen, and Clontech.

### Material and Methods

### 1. Production of VSV-G modified VLPs

The 293T (293ts/A1609) (DuBridge et al., Mol. Cell Biol. 7 (1987). 379-387), 293A (strongly adherent subclone of 293, Quantum Biotechnologies, Montreal, Canada) and S-MAGI cells (Chackerian et al., Virology 213 (1995), 386-394) were maintained in Dulbecco's modified Eagle's medium supplemented with 10 % fetal calf serum (FCS), penicillin, streptomycin and glutamine. The 293T cells were transfected with the calcium phosphate coprecipitation method as described (Soneoka et al., Nucleic. Acids. Res. 23 (1995), 628-633). 5 µg of SX2Δfrxn (Kuate et al., Virology 313 (2003), 653-662) and pHIT-G (Fouchier et al., EMBO 16 (1997), 4531-4539) each were used for transfection of 25 cm² flasks. Two days after transfection, the supernatant of the transfected cells was passed through a 0.45 µm filter and stored in aliquots at -80°C.

### 2. Western blot analysis of VSV-G modified VLPs

The supernatants of 293T cells transfected with the SX2Δfrxn plasmid and pHIT-G as described above was filtered through a 0.45 µm filter and incubated with 293A cells. The supernatant of the exposed cells was harvested 2 days after infection and filtered through a 0.45 µm filter. 5 ml of the filtered supernatants of transfected 293T cells and VLP-exposed 293A cells were pelleted through a 20 % sucrose cushion by ultracentrifugation. Pellets were resuspended in 50 µl of protein loading buffer, and 15 µl were loaded on a 10 % polyacrylamide gel to separate particle associated proteins. After blotting onto Immobilon-P membranes (Millipore, Bedford, USA) according to standard procedures, SIV antigens were detected with a 1:1000 dilution of the plasma of an SIV-infected rhesus monkey (S1604) and a horseradish peroxidase-conjugated, anti-human IgG secondary antibody (Dako, Glostrup, Denmark) followed by DAB staining.

### 3. Animal experiments

Animals were housed at the German Primate Center in Gottingen. Handling of the monkeys and collection of specimens were performed according to institutional guidelines as described previously (Stahl-Hennig et al., AIDS 4 (1990), 611-617). 2 rhesus monkeys of Indian origin which were seronegative for SIV, D-type retroviruses, and STLV-1 were injected subcutaneously with 10 ml of VSV-G modified VLPs at week 0, 4, 8, 29, 33, 37 and 65. The total volume of 10 ml was injected at 3 different sites. The VSV-G modified VLPs were generated by transient cotransfection of 293T cells in 182 cm² tissue culture flasks with 40 µg each of SX2Δfrxn and pHIT-G. 1 day after transfection, the FCS-containing medium was replaced by serum-free AIM-V medium (Gibco Invitrogen Corp., Karlsruhe, Germany), and supernatants were collected and filtered 24 hours later as described above. The amount of reverse transcriptase of the VSV-G modified VLPs was determined by the RT easy kit (Roche Diagnostics, Mannheim, Germany). An amount of approximately 1.6 µg RT was injected at each time point.

For comparative reasons 2 monkeys were also immunized at the time points given above with 10⁷ infectious units of single cycle immunodeficiency viruses (SCIVs) containing 1.35 to 1.88 µg reverse transcriptase and prepared as described (Kuate et al., Virology 313 (2003), 653-662), and a DNA vaccine formulated in cationic lipids as described (DC-high in Nchinda et al., BMC Biotechnol. 2 (2002), 12). The plasmid complexed with the lipids was SX2Δfrxn. All immunized animals were challenged by the tonsillar route 4 weeks after the last booster immunization along with 2 control monkeys with cell-free pathogenic SIVmac239 (Stahl-Hennig et al., Science 285 (1999), 1261-1265).

Antibodies to the SIV polypeptides Env and Gag were determined by ELISA as described (Stahl-Hennig et al., Virology 186 (1992), 588-596). Instead of whole lysed SIV antigen, recombinant SIV p27 kindly provided by the program "European Vaccination against AIDS" (Program EVA) of the European Union was used. For the T-cell proliferation assay, PBMCs were stimulated with 0.25 µg purified baculovirus-derived SIV Gag-VLP (Notka et al., Biol. Chem. 380 (1999), 341-352) for 7 days and ³[H]-thymidine incorporation was measured after a 6 hour incubation period (Hoch et al., J. Virol. 69 (1995), 4807-4813). The stimulation index was calculated from the mean counts of triplicate wells by dividing the mean counts of antigen-containing cultures by the mean counts of cultures without antigen. The T-helper cell proliferative response of an animal was considered positive if stimulation indices over 3 were obtained at least at 2 time points. Quantitative RT-PCR was used to determine the viral RNA load in plasma using standard procedures.

### Results

**1. Preparation of VSV-G modified VLPs**
   Cotransfection of 293T cells with SX2Δfrxn and pHIT-G plasmids led to production of VLPs that could be pelleted through a 20 % sucrose cushion. Western Blot analysis of the VLPs and SCIVs prepared revealed the presence of SIV-Gag and Env proteins (Fig. 2). Incubation of 293A cells with the VLPs did not lead to expression of SIV proteins in 293A cells indicating that the VLPs cannot undergo even a single round of infection.
**2. Immunization of rhesus monkeys with VSV-G modified VLPs**
   2 rhesus monkeys were immunized subcutaneously with VSV-G modified VLPs containing approximately 1.6 µg of reverse transcriptase per injection. Monkeys were immunized on week 0, 4, 8, 29, 33, 37 and 65 (Fig. 3). For comparative reasons, 2 monkeys each were also immunized with single cycle immunodeficiency viruses (SCIVs) and the SX2Δfrxn plasmid formulated in cationic lipids. 2 other monkeys served as untreated controls.
   Antibody titers to SIV capsid protein were determined in the vaccine groups on week 0, 10, 31 and 39. The highest antibody titers were obtained in monkeys immunized with VSV-G-modified VLPs (Fig. 4), and a very poor antibody response was observed in the lipid-DNA vaccinated monkeys. Cellular immune responses were assessed by a SIV-specific T-helper cell proliferation assay. A positive T-helper cell proliferation was observed in both VLP-immunized monkeys and one each of the SCIV and lipid-DNA immunized monkeys.
**3. Challenge of monkeys with SIVmac239**
   4 weeks after the last immunization the monkeys were challenged with SIVmac239 by the tonsillar route. Determination of the viral RNA load in the monkeys after challenge revealed that all monkeys became infected (Fig. 5). However, the SCIV-immunized monkeys and the VLP-immunized monkeys were able to control virus replication. Most strikingly and completely unexpected, both VLP-immunized monkeys cleared viral RNA from the plasma starting 4 to 6 weeks after challenge and maintained undetectable viral RNA levels for the entire observation period.
   These results demonstrate that enveloped VLP vaccines carrying a heterologous protein, which enhances binding, uptake, intracellular processing and/or presentation of the VLP, efficiently induce an antiviral immune response which controls virus replication. Of note, vaccination with non-infectious virus particles lacking a heterologous protein did not lead to protection, as was demonstrated by many groups (Daniel et al., Retroviruses 10 (1994), 839-851; Norley et al., Intervirology 45 (2002), 267-274; Lifson et al., J. Med. Primatol. 31 (2002), 205-216; Lu et al., AIDS 12 (1998), 1-10; Goldstein et al., J. Med. Primatol. 23 (1994), 75-82; Putkonen et al., J. Med. Primatol 22 (1993) 100-103; Le Grand et al., Vaccine 10 (1992), 873-879; Chan et al., J. Exp. Med. 176 (1992), 1203-1207; Stott, Nature 353 (1991), 393). Surprisingly, monkeys immunized with VSV-G modified VLPs cleared the virus more rapidly than monkeys immunized with single cycle immunodeficiency virus (SCIV) vaccines. This was unexpected, since the SCIVs were tought to increase immunogenicity by expression of viral genes in the vaccinees (US 6,500, 623 B1; Kuate et al., Virology 313 (2003), 653-662). However, a direct comparison failed to reveal an increased immunogenicity of the single cycle immunodeficiency virus vaccines (Fig. 3 and 4).
   The VSV-G modified enveloped VLPs also compare favorable to other viral vector vaccines and DNA vaccines. For such a comparison it is important to assess the stringency of the challenge system. Similar to primary isolates of HIV-1, the SIVmac239 used in this study is highly resistant to neutralization (Langlois et al., J. Virol. 72 (1998), 6950-6955; Montefiori et al., J. Immunol. 157 (1996), 5528-5535) and it leads to a slow and inexorable CD4 destruction (Lewis et al., AIDS Res. Hum. Retroviruses 10 (1994), 213-220; Luciw et al., AIDS Res. Hum. Retroviruses 8 (1992), 395-402). Thus, it is one of the most difficult viruses to protect from in the SIV macaque model (Horton et al., J. Virol. 76 (2002), 7187-7202). Therefore, it is a great achievement of the present inventors to develop a protective VLP based vaccine, which efficiently protects subjects from the challenge with the lethal virus, while entirely avoiding the risk of an insertional mutagenesis. With the exception of live-attenuated SIV vaccines (reviewed in Desrosiers, HIV Adv. Res. Ther. 3 (1996), 3-9; Johnson, Nat. Med. 5 (1999), 154-155), which are impossible in humans due to safety issues, all other vaccine approaches including DNA vaccination and combined vaccination with DNA and modified vaccinia virus Ankara (MVA) failed if the SIVmac239 challenge virus was used (Wang et al., J. Virol. 74 (2000), 10514-10522; Horton et al., J. Virol. 76 (2002), 7187-7202) underlining the striking protective effect of VSV-G modified VLPs.

### References

Amara et al., Science 292 (2001), 69-74
Baba et al., Science 267 (1995), 1820-1825
Baba et al., Nat. Med. 5 (1999), 194-203
Baker et al., Biophys. J. 60 (1991), 1445-1456
Barouch et al., Immunol. Lett. 79 (2001), 57-61
Chackerian et al., Virology 213 (1995), 386-394
Chan et al., J. Exp. Med. 176 (1992), 1203-1207
Daniel et al., AIDS Res. Hum. Retroviruses 10 (1994), 839-851
Desrosiers, HIV Adv. Res. Ther. 3 (1996), 3-9
DuBridge et al., Mol. Cell Biol. 7 (1987), 379-387
Fouchier et al., EMBO 16 (1997), 4531-4539
Gabuzda et al., J. Virol. 66 (1992), 6489-6495
Giavedoni et al., J. Virol. 67 (1993), 577-583
Goldstein et al., J. Med. Primatol. 23 (1994), 75-82
Habel et al., Dev. Biol. (2000), 101-105
Hacein-Bey-Abina et al., Science 302 (2003), 415-419
Hagensee et al., J. Virol. 68 (1994), 4503-4505
Hoch et al., J. Virol. 69 (1995), 4807-4813
Horton et al., J. Virol. 76 (2002), 7187-7202)
Hu et al., Science 255 (1992), 456-459
Hu et al., J. Med. Primatol. 21 (1992), 119-125
Johnson, Nat. Med. 5 (1999), 154-155
Kochetov et al., FEBS Letts. 440 (1998), 351-355
Kuate et al., Virology 313 (2003), 653-662
Langlois et al., J. Virol. 72 (1998), 6950-6955
Le Grand et al., Vaccine 10 (1992), 873-879
Lewis et al., AIDS Res. Hum. Retroviruses 10 (1994), 213-220
Lifson et al., J. Med. Primatol. 31 (2002), 205-216
Lu et al., AIDS 12 (1998), 1-10
Luciw et al., AIDS Res. Hum. Retroviruses 8 (1992), 395-402
Luciw et al., PNAS USA 92 (1995), 7490-7494
Lund et al., J. Virol. 71 (1997), 1191-1195
Malenbaum et al., J. Virol. 74 (2000), 11008-11016
McCaughan et al., PNAS USA 92 (1995), 5431-5435
Montefiori et al., J. Immunol. 157 (1996), 5528-5535
Mooij et al., AIDS 12 (1998), F15-F22
Naldini et al., Science 272 (1996), 263-267
Nchinda et al., BMC Biotechnol. 2 (2002), 12
Norley et al., Intervirology 45 (2002), 267-274
Notka et al., Biol. Chem. 380 (1999), 341-352
Piguet et al., Immunol. Rev. 168 (1999), 51-63
Planelles et al., J. Virol. 70 (1996), 2516-2524
Putkonen et al., J. Med. Primatol 22 (1993), 100-103
Sanchez-Pescador et al., Science 227 (4686) (1985), 484-492
Sheehey et al., Nature 418 (2002), 646-650
Shiver et al., Nature 415 (2002), 331-335
Soneoka et al., Nucleic. Acids. Res. 23 (1995), 628-633
Stahl-Hennig et al., AIDS 4 (1990), 611-617
Stahl-Hennig et al., Science 285 (1999), 1261-1265
Stahl-Hennig et al., Virology 186 (1992), 588-596
Stott, Nature 353 (1991), 393
Stott et al., J. Gen. Virol. 79 (1998), 423-432
Wang et al., J. Virol. 74 (2000), 10514-10522

US Patent No. 6,500,623
US Patent No. 5,156,949
US Patent No. 5,688,688
EP Patent No. 0 570 980

## Claims

1. Use of a virus-like particle (VLP) for the preparation of a composition for immunization against an immunodeficiency virus, wherein said VLP is formed by at least a part of the viral protein Gag, said VLP comprises an envelope with at least one viral protein or a part thereof and at least one heterologous protein, said VLP is replication defective, and the at least one nucleic acid coding for said VLP is deleted in at least one open reading frame coding for the viral proteins Vif, Vpx, Vpr, Vpu, Nef, Rev, Tat, Pol, and/or Env or parts thereof.

2. The use of claim 1, wherein said immunodeficiency virus is the human immunodeficiency virus (HIV).

3. The use of claim 1 or 2, wherein said viral protein forming said VLP is selected from the group consisting of Gag, Pol, Env, Tat, Rev, Nef, Vif, Vpr, Vpx and Vpu.

4. The use of any of the preceding claims, wherein said viral protein is mutated or modified to increase immunogenicity and/or representation of antigenic variants of HIV.

5. The use of any of the preceding claims, wherein said replication deficiency is mediated by a mutation in the primer binding site of said immunodeficiency virus.

6. The use of any of the preceding claims, wherein said heterologous protein is the vesicular stomatitis virus G-protein (VSV-G).

7. The use of any of the preceding claims, wherein said heterologous protein is an antibody.

8. The use of claim 7, wherein said antibody is a single chain antibody targeting a specific human cell surface receptor.

9. The use of any of the preceding claims, wherein said heterologous protein is a ligand or modified ligand of a specific human cell surface receptor.

10. A pharmaceutical composition comprising a virus-like particle (VLP) for immunization against an immunodeficiency virus, wherein said VLP is formed by at least a part of the viral protein Gag, said VLP comprises an envelope with at least one viral protein or a part thereof and at least one heterologous protein, said VLP is replication defective, and the at least one nucleic acid coding for said VLP is deleted in at least one open reading frame coding for the viral proteins Vif, Vpx, Vpr, Vpu, Nef, Rev, Tat, Pol, and/or Env or parts thereof.


SEQUENCE LISTING

<110> Ruhr-Universität Bochum

<120> Virus-like particle (VLP) as vaccine

<130> R30001

<140> EP03028826.0
<141> 2003-12-15

<160> 1

<170> PatentIn version 3.1

<210> 1
<211> 18
<212> RNA
<213> Artificial Sequence

<220>
<223> Primer binding site (PBS) X2

<400> 1
uggucaguug caggggau